# EUROPEAN PATENT SPECIFICATION

(11) **EP 4 243 702 B1**
(45) Date of publication and mention of the grant of the patent: **14.05.2025**
(21) Application number: 20806997.1
(22) Date of filing: 12.11.2020
(51) Int. Cl.: A61B 17/062, A61B 17/06, A61B 17/04, A61B 90/00, A61B 17/00

(54) **A SURGICAL DEVICE AND SYSTEM**
CHIRURGISCHE VORRICHTUNG UND SYSTEM
DISPOSITIF CHIRURGICAL ET SYSTÈME

(43) Date of publication of application: 20.09.2023
(73) Proprietor: ZuriMED Technologies AG, 8008 Zürich (CH)
(72) Inventor: BACHMANN, Elias, 8006 Zürich (CH); LI, Xiang, 8126 Zumikon (CH); BANZET, Pol, 8008 Zürich (CH)
(74) Representative: Vossius & Partner Patentanwälte Rechtsanwälte mbB
(86) International application number: PCT/EP2020/081887
(87) International publication number: WO 2022/100833

(56) References cited:
- EP-A1- 3 066 999
- WO-A1-2016/044762
- US-A1- 2010 145 306
- US-A1- 2015 231 368
- US-A1- 2017 312 079
- US-B2- 8 454 494

## Description

The present invention relates to a surgical device, a system, and a method of manufacturing a surgical device according to the preambles of the independent claims.

The present applicant recently developed a surgical felting device allowing a biomechanically advantageous implantation of implants. The developed device allows an improved fixation as compared to conventional suturing techniques. One example of such a surgical felting device is disclosed in PCT/CH2019/000015. The surgical felting device comprises a needle that repeatedly moves through a surgical felt and into tissue. By embedding strands of the felt inside the tissue, the needle creates a strong and well distributed mechanical bond between the felt and the tissue. Compared to conventional suturing, this technique is faster and alleviates adverse effects such as *"cheesewiring"* of suture, where the suture cuts through tissue which can happen through local stress peaks.

However, since the surgical felting device may be handheld and the needle moves at high speeds, there is high risk of needle damage. The damage may result from collisions between the needle and rigid structures such as bones or other surgical tools. Such collisions could lead to a partial destruction of the needle (plastic bending, breaking or splintering) which adversely influences the functionality. Even further, parts of the needle may be lost inside the patient's body, presenting a health risk. Lastly, the high speeds of the needles in the tissue and in the surgical felt may generate undesirable heat that results from friction.

US 2015/231368 A1 discloses a needle-injection catheter that includes a catheter body having a distal end, a proximal end, a stiff proximal portion, a flexible distal portion, and a delivery lumen extending therethrough. In a first embodiment, a straight injection needle extends coaxially from a distal tip of the flexible portion of the catheter body, and a plurality of penetration limiting elements positioned circumferentially about a base of the straight injection needle and configured to fold radially inwardly against a shaft of the needle when constrained in a tubular lumen and to extend radially outwardly when unconstrained. In a second embodiment, a helical needle extends from the distal tip of the flexible portion of the catheter body. The helical needle has at least one helical delivery lumen connected to receive an injectable substance from the delivery lumen of the catheter body.

EP 3 066 999 A1 discloses a catheter system that includes a positioning catheter for receiving an injection needle into its lumen. Structurally, the injection needle incorporates a plurality of loops that are mounted directly onto its shaft. As the injection needle is moved in a distal direction to exit from the lumen of the catheter, the loops are individually biased to transition from a folded configuration, and into a deployed configuration. In their deployed configurations, the loops create a barrier that is oriented perpendicular to the needle. Thus, the barrier acts to limit the depth of insertion of the needle into target tissue of a patient, to a predetermined depth, and to prevent perforation of the target tissue by the catheter tip.

US 8,454,494 B2 discloses a support apparatus connects with and provides fluid communication between a needle and a syringe. The support apparatus includes at least one arm that is configured to provide cantilever support of the needle and syringe while the needle is inserted in a patient. The arm engages the patient's skin, and may be rigid, malleable, hinged, stretchable, telescoping, and/or have other properties. The support apparatus may form part of an adapter that also includes a pressure sensor. The pressure sensor senses the pressure of fluid in a gastric band system when the needle is inserted in an injection port of the gastric band system. A cable may extend from the pressure sensor to a display device, and may include markings configured to provide measurement from the needle to a patient's xyphoid process. Such a measurement may be factored into a calculation to account for hydrostatic pressure differences in pressure readings.

US 2010/145306 A1 discloses a catheter device comprising a proboscis shaft and a proboscis disposed within the proboscis shaft. In certain embodiments, the catheter device further comprises an elongate tubular member, wherein the proboscis shaft is disposed within the elongate tubular member. In certain embodiments, the catheter device comprises a tissue surface engagement structure, which has a first configuration and a second configuration. In the second configuration, the tissue surface engagement structure presents a larger transverse profile in comparison to the first configuration. The tissue surface engagement structure may have any of various designs, including an expandable assembly and a hinged assembly. In certain embodiments, a deformable cushion is positioned at the distal end of the proboscis shaft. The deformable cushion comprises a pocket that is filled with a reshapeable material. In certain embodiments, the proboscis shaft comprises a longitudinally compressible portion.

It is the objective technical problem to overcome the above disadvantages of the prior art. In particular, it is the objective technical problem to provide a surgical felting device that is safe to use for the operator and the patient. A further object of the present invention may be to reduce the heat generated by the felting needle.

The objective technical problem is solved by the features of the independent claims. One aspect of the invention relates to a surgical device for felting an implant to soft tissue of a patient. The patient may be a human or an animal. Felting may be understood as the process of moving a felting needle repeatedly into, and in some embodiments through, an implant comprising a felt and thereby entangling fibers of a felt. A felting needle may be understood as a needle including barbs that catch the fibers of the felt and move the fibers through the felt and which entangles the fibers of the felt further. In particular, the felting may not only entangle the fibers of a felt that forms part of an implant with each other but also entangle the fibers of the tissue of a patient with the fibers of the felt. Thereby, some strands of the felt become embedded in and entangled with the patient's tissue and some strands of the tissue fibers may become embedded in and entangled with the felt. This creates a strong and well distributed mechanical bond between tissue and felt.

The present invention relates to a surgical device for felting an implant to soft tissue of a patient as defined by independent claim 1. Preferred embodiments are given in the dependent claims.

The surgical device comprises at least one felting needle. The at least one felting needle is configured to move reciprocally. A reciprocating motion as mentioned herein may be understood to be a repetitive back and forth motion. The motion may be translational, i.e. linear, or rotational. Further, the surgical device comprises a connection interface for connecting the at least one felting needle to an actuator and for transferring a reciprocal motion from the interface to the at least one felting needle. The interface may provide a releasable or a permanent attachment. For example, the releasable interface may include pins, latches, screws, magnets or other known mechanical or other connection means. A permanent attachment using rivets, glue or a one-piece design is also possible. A releasable attachment of the at least one needle would allow the at least one needle to be replaced.

In certain embodiments the device includes a water cooling system to cool down friction heat which is generated by the oscillating needle.

The actuator may be pneumatic, hydraulic or electromagnetic. In particular, the actuator may be an electric motor or may comprise or use electromagnetic coils or pneumatic or hydraulic pressure. The device may be configured to convert a rotational motion of the actuator to a translational motion. In particular, the device may comprise a scotch yoke mechanism or a piston rod drive mechanism to transform a rotational motion into a translational motion. Thereby, more efficient motors may be used. A speed and/or frequency of the reciprocal translational motion (oscillation) can be adjustable. The device may comprise a linear motion rod to transfer the reciprocal motion from the interface or the actuator to the at least one needle. To reduce wear and heating, bearings and low friction materials (e.g. polytetrafluorethylene) may be used to minimize friction of a linear motion rod.

The at least one needle is preferably made of or comprises stainless steel. The needle may include a stainless steel coating. Stainless steel is particularly resistant to the oxidizing conditions of the surgical operation. Hence, the present device may e.g. be used in arthroscopic surgeries, in which a constant saline flush is applied, that may oxidize the needle.

The device comprises a needle protection mechanism. The needle protection mechanism prevents the at least one needle from being damaged during the reciprocal motion due to a contact with a rigid structure.

The needle protection mechanism may define a maximal penetration depth of the at least one needle. The maximal penetration depth may be adjustable. In particular, the maximum penetration depth may be less than the amplitude of the reciprocal motion. Additionally or alternatively, the needle protection mechanism may limit the reciprocal motion or may be configured to detect an obstacle (i.e. hard tissue) or may decouple an actuator from the at least one needle in case a collision occurs or any combination thereof. The motion may be limited by modifying the amplitude of the reciprocal motion and/or by limiting the maximal penetration depth.

The rigid structure may be for example the bone of a patient or another surgical tool that may come in contact the needle while the needle is actuated.

The surgical device may be used for the repair or fixation of anatomical structures such as: collagenous tissues such as tendons, menisici, spinal disks or fascia, ligament reconstructions (collateral ligaments, cruciate ligaments, etc.), subcutaneous sutures, conventional suturing of skin closures, skeletal muscle, heart muscle, valves, and hollow organs (large vessels, bladder, esophagus, possibly intestine). In addition, an implant may be attached to these anatomical structures.

In one embodiment, the at least one felting needle has a width or diameter of less than 0.8 mm, preferably less than 0.6 mm, most preferably less than 0.4 mm. Needles with a low diameter have a lower friction and thus less heat is generated. However, the lower needle diameters may lead to more fragile needles.

The needle protection mechanism includes a spacer for contacting the soft tissue or patch such that the spacer sets a maximal predetermined penetration depth for the at least one needle. The spacer pushes against the tissue and thereby prevents the needle from penetrating further than desired. This may be especially useful for anatomical structures that are arranged close to bones such as tendons or spinal disks. Further, the spacer helps an operator to keep the amplitude of the motion of the needle within a desired range (e.g. the felt and the soft tissue) and improves handling.

The spacer may be elastic, in particular, the spacer may include or form a spring. Elastic may be understood as allowing the user to press the device against the soft tissue and thereby changing the spacing of the spacer dependent on the applied force. In one embodiment the spacer may have a curved or bent shape. The elasticity allows a constant contact of the tip of the device on the feltable patch or tissue. Further, this may help holding the soft tissue in place without the need for further tools and/or assistance. If there is no constant contact to the tissue or patch, the tissue or patch could vibrate and reduce a penetration of the needle through the tissue. In particular, the spacer prevents the needle from lifting the patch, when the needle is retracted (stripping effect). When the needle is retracted from the felt and/or the tissue, the barbs and/or friction may pull the patch towards the surgical device. The spacer prevents this and the at least one needle will pull out of the patch and/or the tissue without lifting it.

In a preferred embodiment, the spacer comprises one or more fingers. Preferably, the spacer comprises two, three or more fingers. The one or more fingers comprise a distal end surface for contacting the soft tissue such that the reciprocal motion of the needle does not exceed the predetermined penetration depth. The one or more fingers may be bent at their distal side such that a radial outer surface of the fingers forms the distal end surface(s) of the spacer. Thereby, the operator may visually observe the at least one felting needle allowing for a more precise control of the device. Further, the bent fingers may allow an elasticity of the fingers.

In some embodiments, the fingers may be connected to each other at their distal side, and in particular be formed by a single wire. The fingers may be made from a shape memory-alloy, e.g. nitinol. The fingers may have a compressed and expanded configuration. For example the fingers may be expelled from a distal end of a guide tube for the needle.

In a preferred embodiment, the spacer forms a slide such that the needle can be slid along a surface of the soft tissue. In particular, the one or more fingers may be bent in the same direction. The device may then be moved over the soft tissue in the direction in which the curvature points with ease while at the same time maintaining a constant penetration depth and applying a constant pressure keeping the soft tissue in place. The slide may alternatively be formed similarly to slides known from sewing machines.

In a preferred embodiment, the at least one felting needle has a maximal penetration depth. The maximal penetration depth may be adjustable. Thereby, the device may be adapted to the particular use case. Further, an operator may adjust the penetration depth in case the operation is close to hard tissue.

In a preferred embodiment, the reciprocal motion has an amplitude. The amplitude may be adjustable. Thereby, the device may be adapted to the particular use case. Further, an operator may adjust the maximal penetration depth by adjusting the amplitude in case the operation is (too) close to hard tissue.

In a preferred embodiment, the device comprises a guide tube. The guide tube surrounds the at least one needle and/or a translation rod at least partially. The translational rod may transfer the reciprocal motion from the interface to the at least one needle. The guide tube may be an outer tubular member. Further the guide tube may be connected to a spring in series or may be elastic.

In a preferred embodiment, the device may comprise a scale indicating the current penetration depth. In a preferred embodiment, the device may comprise a scale indicating the current maximal penetration depth.

In a preferred embodiment, the needle protection mechanism comprises a needle collision sensor. In a preferred embodiment, the collision sensor is configured to measure the distance to a rigid structure. The needle collision sensor may be a distance sensor, such as for example an ultrasonic sensor. The sensor may measure a distance between the device, in particular a housing or the guide tube, and a hard tissue. Thereby, hard tissue or other obstacles, that may damage the needle can be detected. Additionally, the device may comprise an indicator for indicating the sensed distance to a hard tissue or another obstacle to the operator. The indicator may be optical (i.e. a display or a diode) or haptical or acoustic.

In a preferred embodiment, the needle collision sensor is configured to measure a bending of the at least one needle and/or the linear motion rod. The sensor may be an elastic strain sensor and/or an electromagnetic sensor and/or piezoelectric sensor. When the at least one needle collides with hard tissue such as bone, the needle and/or linear motion rod may bend before breaking. The needle collision sensor detects the bending. This may cause a controller to decouple the needle from the actuator or changes to reduce the maximal penetration depth.

In a preferred embodiment, the surgical device comprises a controller. The controller is configured to receive a signal from the sensor and adjust the maximal penetration depth of the at least one needle based on the measurement of the needle collision sensor. Thereby an open control loop is provided allowing for a quick, automatic adjustment of the penetration depth in response to the detection of a collision or the detection of the threat of a collision. In some embodiments, the actuator may measure the current maximal penetration depth with a further sensor and the controller may be configured to receive a signal from the actuator for a closed loop control of the maximal penetration depth.

The surgical device may comprise an actuator for driving the reciprocal motion of the at least one needle. The surgical device comprises a coupling adapted to transfer a reciprocal motion from the actuator to the at least one needle. The coupling may be a slipper clutch. A slipper clutch may be understood herein as a clutch that decouples the needle from the actuator, when a threshold force or threshold moment is exceeded. The slipper clutch may slip at least partially when a predetermined force or moment acting on the at least one needle is exceeded. The needle protection mechanism is thereby adapted to decouple the needle from the actuator to prevent the needle from being damaged when a collision occurs.

In a preferred embodiment, the slipper clutch includes at least one of: a pin and a chamfered face, a ball spring and a corresponding dent for receiving the ball and a spring bearing for the at least one needle.

In a preferred embodiment, the needle protection mechanism includes a predetermined breaking point. The predetermined breaking point breaks, if the force on the at least one needle exceeds a predetermined threshold. The predetermined threshold may be lower than the force necessary to break the needle.

In a preferred embodiment, the predetermined breaking point is part of a mechanical coupling for transferring forces onto the at least one needle. The mechanical coupling preferably includes a rod having the predetermined breaking point. In some embodiments, the predetermined breaking point may be provided by a structural weakening such as a tapering, or a ridge, an edge or a dent. In other embodiments, the predetermined breaking point is part of the at least one needle.

In a further embodiment, the device is configured to detect a broken needle. In particular the needle may be part of the electric circuit or may include a needle collision sensor as described above. Further the device may include an indicator, e.g. a warning light, that is configured to indicate optically, haptically or acoustically that the needle is broken.

A further aspect of the invention relates to a system comprising a surgical device as described above and an actuator. The actuator may be in particular an electric motor for driving the at least one needle with the reciprocal motion.

Non-limiting embodiments of the invention are described, by way of example only, with respect to the accompanying drawings, in which:
- Figure 1:: shows a schematic perspective view or a first embodiment of a surgical device according to the invention.
- Figures 2A and 2B:: show further details with regard to an adjustable maximal penetration depth of the surgical device according to figure 1.
- Figures 3A and 3 B:: show alternative embodiments for a distal tip of the surgical device according to figure 1.
- Figure 4:: shows a schematic perspective view of a second embodiment of a surgical device according to the invention.
- Figure 5:: shows a cross-section of the surgical device according to figure 4.
- Figures 6A and 6B:: show an interface for the actuator of a surgical device according to the invention.
- Figure 7A and 7B:: show a further interface for the actuator of a surgical device according to the invention.
- Figure 8:: shows a cross-section of a distal portion of a third embodiment of a surgical device according to the invention.
- Figure 9:: shows a cross-section of a distal portion of a fourth embodiment of a surgical device according to the invention in a first position.
- Figure 10:: shows a cross-section of a distal portion of the surgical device of figure 9 in a second position.
- Figure 11:: shows a cross-section of a distal portion of a fifth embodiment of a surgical device according to the invention.

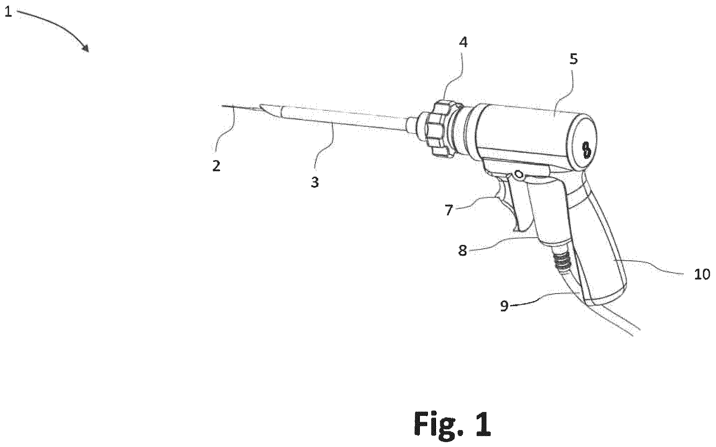

Figure 1 shows a perspective view of a first embodiment of a surgical device 1 according to the invention. The surgical device 1 includes a housing 5 and a guide tube 3 extending from the housing 5. A felting needle 2 is arranged within the guide tube 5 and extends from the end of the guide tube of 5. The felting needle 2 can be moved reciprocally forwards and backwards along its axis and the axis of the guide tube 3. The surgical device 1 includes a handle 10 with a grip portion 7. Further, the surgical device 1 is connected to an energy source with a power cord 9 that connects to the surgical device.

An actuator -here electrical motor 8 - is arranged within the housing 5 of the surgical device 1. The motor 8 drives the reciprocal motion of the needle 2. The guide tube 3 may be moved with respect to the needle 3 using wheel 4. When the wheel 4 is turned, the guide tube 3 is retracted in a distal direction such that the tip of the needle 2 is exposed. When the wheel 4 is turned in an opposite direction, the guide tube 3 may be pushed in a proximal direction such that the tip of the needle 2 can be covered. Consequently, a penetration depth of the needle of the reciprocal motion of the needle is dependent on the relative position of the guide tube. If, for example, the needle 2 has a movement amplitude of 25 mm but the guide tube is only retracted by 10 mm from the most distal end position of the needle during the reciprocal motion, then the maximal penetration depth of the needle is only 10 mm. Proximal and distal are understood herein from the perspective of an operator, i.e. distal denotes a direction away from the operator and proximal a direction towards the operator.

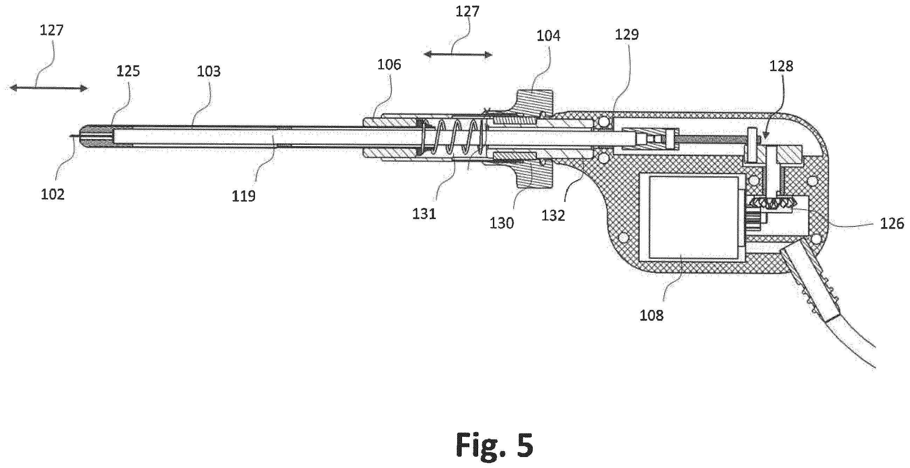

Setting the guide tube 3 relatively to the needle 2 limits the maximal penetration depth of the needle and allows an operator to avoid felting tissues that are deep below the implantation site and in particular to avoid collisions with hard tissues such as bone. Further, the guide tube 5 protects the needle 2 during transportation and unpacking of the device. The guide tube 5 is embedded in a guide plug 6 (see fig. 1) and may be connected in series to a spring (see e.g. fig. 5).

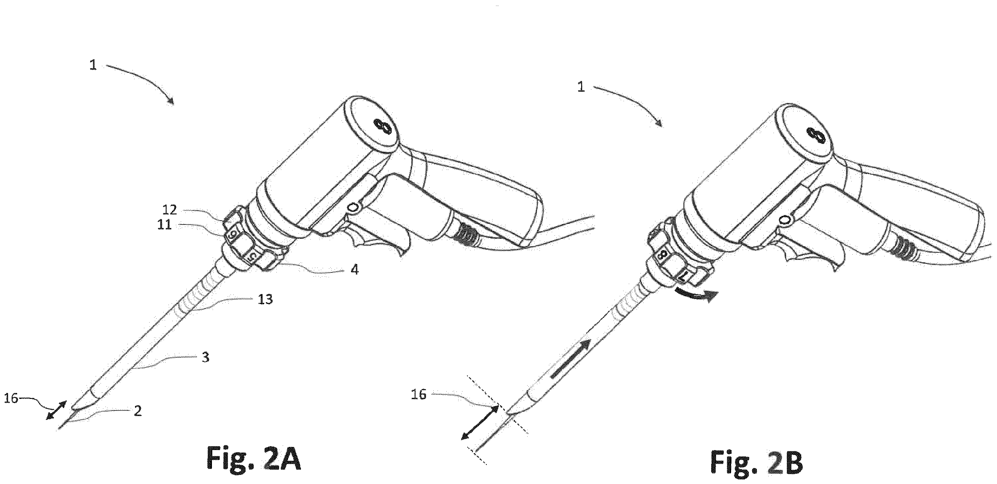

Figure 2A shows the relative movement of the guide tube 3 in further detail. The wheel 4 includes ridges 12 such that an operator can grip the wheel more easily. Further, the wheel includes indicators 11 that show the current position of the guide tube 3. In the shown example, the number on the wheel indicates a length of the needle that can maximally be exposed, i.e. a maximal penetration depth. Further, the guide tube 3 itself includes a penetration depth scale 13. The penetration depth scale 13 is realized as markings on the outer surface of the guide tube 3. In the present example, the markings are circular rings around the guide tube 3. When the guide tube 3 is retracted, the current penetration depth can be read from the last ring that is still visible, i.e. not retracted into the housing 5. The depth scale shows the current penetration depth. In case the guide tube 3 is directly retracted as a result of the movement of the wheel 4, the current and maximal penetration depth are the same. However, in some embodiments these two may differ as will be explained with reference to the second embodiment.

The relative movement of the guide tube 3 is illustrated by the arrows in figs. 2A and 2B. If the operator turns the wheel counterclockwise, the guide tube 3 is retracted, exposing the indicated maximal penetration depth 16.

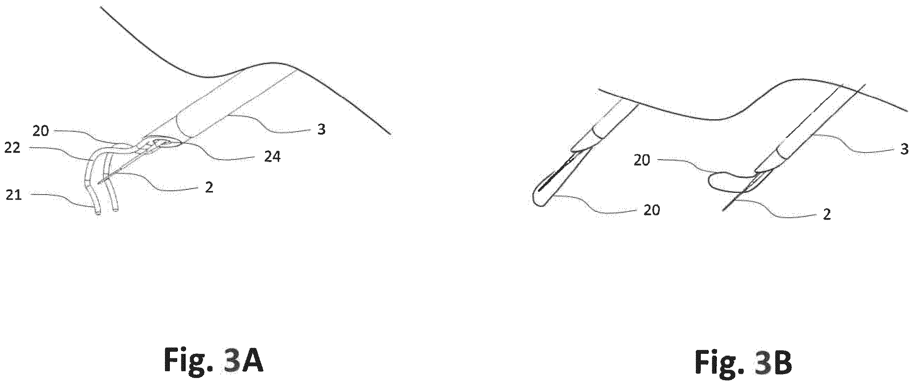

Figures 3A and 3B show further embodiments of the guide tube 3 that may be used independently or in combination with the retractable guide tube 3. In the embodiment of figure 3A, the guide tube 3 comprises at its distal end 24 a protective fork 20 with two fingers 21. The fingers 21 extend from the distal end and are bent around a curve 22. During use, the distal end surfaces of the fingers 21 are brought in contact with a soft tissue of a patient and define the penetration depth 16 similarly to the end of the guide tube 3 in the previously shown embodiment. The curvature of the fingers 21 allows the surgical device to slide smoothly over the soft tissue by helping the surgical device 1 to glide along the felt and/or soft tissue that are currently felted.

In the embodiment of figure 3B, the protective fork is formed by a wire made of a shape memory alloy. During transportation, the wire is held within the guide tube. Prior to an operation, the wire is expelled from the distal end 24 of the guide tube and assumes a bent position as shown on the right side in figure 3B.

In case an operator pushes in a distal direction, the bends wire or the bent fingers may act as a spring allowing to temporarily (depending on the force) increase the penetration depth if necessary.

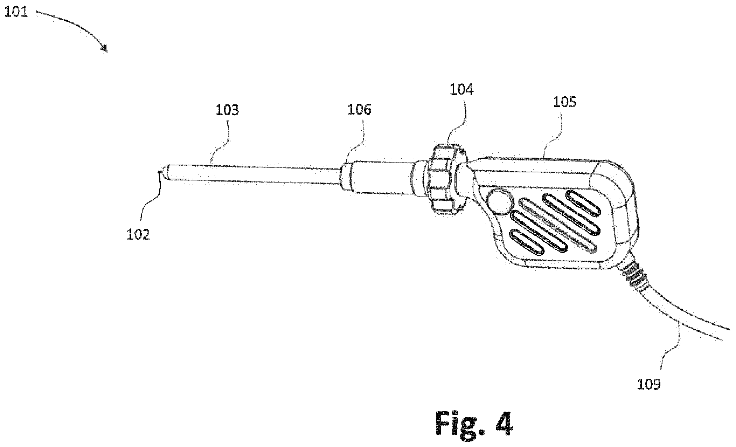

Figures 4 and 5 show a second embodiment of a surgical device 101 according to the invention. A perspective view of the surgical device 101 is shown in figures 4 and a cross-section of the surgical device 101 is shown in figure 5. In general, the present description uses similar reference numerals for similar features, when the reference numeral increases by 100. For example, the felting needle 2 may be similar to the felting needle 102 of the embodiment shown in figure 4.

Similarly to the previously shown nrst embodiment, the second embodiment of a surgical device 101 includes a felting needle 102, a guide tube 103, a plug 106, a wheel 104 for retracting the guide tube 103, and a housing 105. A power cord 109 is connected to the housing 105.

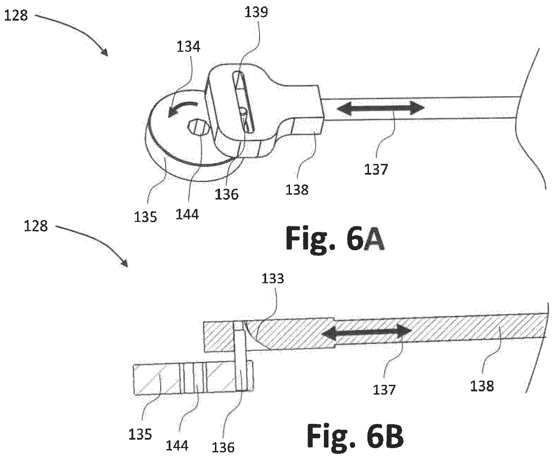

As can be seen from figure 5, the surgical device 101 includes a motor 108. The motor 108 drives an axis whose rotation is then transferred via gear units 126 to a scotch yoke 128. The scotch yoke 128 transforms the rotational motion of the motor 108 into a translational motion. One embodiment of a scotch yoke 128 can be seen in detail in figures 6A and 6B. The scotch yoke 128 transfers the rational movement to a linear movement and the linear movement to translational motion rod 119 that is held within the guide tube 103. The translational motion rod 119 is guided by a bearing 129. The needle 102 is arranged at the distal end of the translational rod 119 and travels back and forth, wherein the amplitude of the movement is defined by the scotch yoke 128 and the frequency of the reciprocal movement is determined by the motor 108.

The surgical device 101 also includes a wheel 104. The wheel 104 includes an inner thread 130 that engages an outer threading of an insert 132. The outer tube 103 covers the translational rod 119 and is covered at its distal end with a distal tip 125. The proximal side and proximal end of the guide tube 3 includes a plug 106. The plug 106 is also connected to a spring 131 along its axial direction. Thereby the spring 131 is arranged between the plug 106 and the insert 132. During operation of the surgical device 101, the operator may push the distal tip with felting needle 102 onto a felt. Thereby, the outer tube 103 is pushed in a proximal direction. The spring 131 resists this movement such that an operator has to push against it. Thereby, the outer tube 103 covers the sharp needle 102, when the device is not in use.

Further, the wheel 104 can be used to move the insert 132 back and forth. The insert delimits a maximum width 127 for the retraction of the outer tube 103. The maximum width 127 for the retraction thus corresponds to a maximal penetration depth of the needle 102. The delimitation of the maximal penetration depth protects the needle 102 from colliding with hard tissue, since the heart tissue (e.g. bony tissue) may be arranged below the soft tissue. Setting the maximal penetration depth as described is advantageous, if different kinds of tissues with hard tissue beneath are felted (e.g. an 8 mm rotator cuff tendon or a 12 mm Achilles tendon).

Besides the protection function, the spring 131 may also allow a constant contact of the tip of the device on the feltable patch, respectively tissue. If there is no constant contact to the tissue or felt, the tissue could vibrate and minimize penetration of the needle through the tissue.

Figures 6A and 6B show an embodiment of the scotch yoke 128 in detail. The scotch yoke may form an interface or a coupling. The scotch yoke 128 includes a wheel 135. The wheel 135 is driven by the motor 108 through the hexagonal socket 144 and rotates around direction 134. On a radially outer part of the wheel, a pin 136 is arranged. Further, the scotch yoke includes a slider 138. The slider 138 includes a slot 139, in which the pin 136 travels. Due to the rotation of the wheel forces in a linear direction along the axis of translational rod 119 are transferred as indicated by arrows 137 while the forces in the direction transversal thereto are not transferred due to the slot 139.

Figure 6B shows an embodiment that shows an example of a mechanical uncoupling between the needle 102 and the motor 108. The slider 138 may include a chamfered face 133 at the slot 139. If excessive forces are applied onto the needle 102, these forces are transferred via the linear motion rod and the slider to the scotch yoke 128. The chamfered face 133 glides onto the pin and thereby lifts the slider 137 out of the pin 136 preventing the application of further forces onto the needle 102. Hence, the wheel 135 and the slider 138 are mechanically decoupled.

In a further example, an electromagnetic motor 108 may be used. In this case, the amplitude of the needle 102 may be adjusted, if the motor detects forces above a threshold on the needle 102. If the forces on needle 102 exceed the electromagnetic forces of the linear drive, the needle may be retracted or, the motor may simply be stopped.

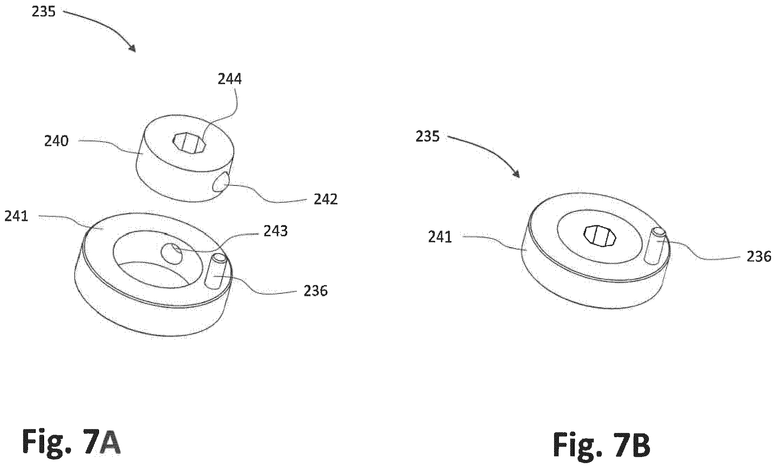

A further mechanical uncoupling is shown in figures 7A and 7B. Figures 7A and 7B show a wheel 235. Wheel 235 is an alternative embodiment of the wheel 135 of figures 6A and 6A. The wheel 235 includes two parts, an inner wheel 240 and a concentric outer wheel 241. Figure 7A shows an exploded view of the wheel 235 and figure 7B shows the wheel 235 in an assembled form. Similarly to the wheel 135, the wheel 235 includes a hexagonal socket 244 and a pin 236. The inner wheel 240 is coupled to the outer wheel with a ball 242. The ball 242 is pushed radially outwardly by a spring (not shown). The outer wheel 241 includes a dent 243 along its inner circumference for the ball 242. When the inner wheel 240 is set into the inner circumference of the outer wheel 241, the ball 242 is pushed radially inwardly against the spring force and latches into the dent 243, if correctly aligned. As long as the ball 242 is in the dent 243, forces are transmitted from the motor 108 to the needle 102. However, in case the needle 102 collides with hard tissue, ball 242 is pushed inwards and no force beyond a threshold are transmitted. Thereby, the two wheels 240 and 241 are rotationally uncoupled.

Alternatively, the ball spring and dent may be replaced by a weak link that would break in case of excessive forces or a latch. A latch could be relying on springs or another compliant mechanism. A further alternative are magnets that may be finely tuned to disengage when needed and re-engage it when the force drops below a threshold. In other embodiments, the system can be electromechanical, with force sensors or other sensors (e.g. strain of the needle, conductivity) detecting that the forces on the needle exceed a threshold. A signal of these sensors may cause a controller to uncouple the needle from the actuator or stop the actuator.

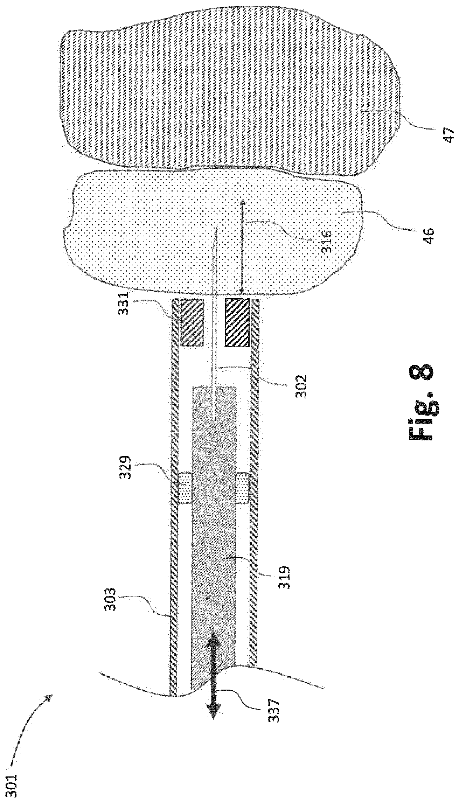

A third embodiment of a surgical device 301 according to the invention is shown in figure 8. The surgical device 301 includes a guide tube 303, and a needle 302 with a maximal penetration depth 316. As can be seen from figure 8, the needle is moved back and forth within soft-tissue 46. The needle 302 is driven by a translational rod 319 with a linear motion 337. The translational rod 319 is guided by bearing 329. Additionally, the surgical device 301 includes an ultrasonic distance sensor 331. The ultrasonic distance sensor 331 is arranged at the distal end of the guide tube and may measure a distance between a distal end of the guide tube 303 and bony tissue 47. The measured distance may be reported to a user with acoustic, optical or vibrational cues that allow the user to set the maximal penetration depth 316. For example, the maximal penetration depth may be set as described with respect to figures 1 to 5. Alternatively, the surgical device 301 may additionally include a controller, that automatically adjusts the maximal penetration depth 316 to be less than the measured distance.

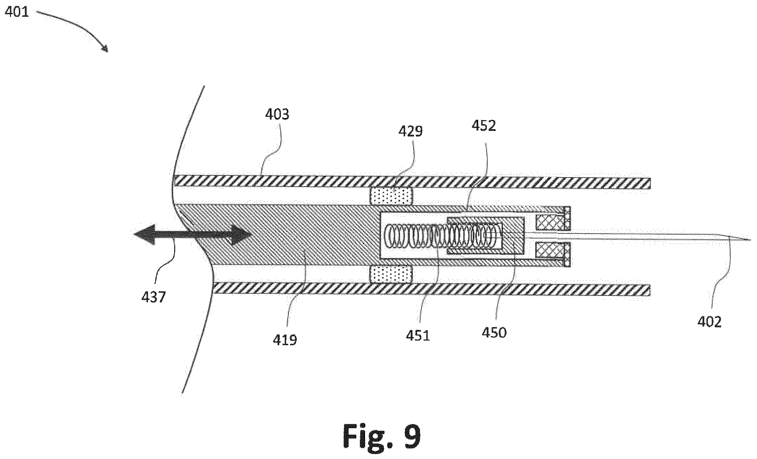

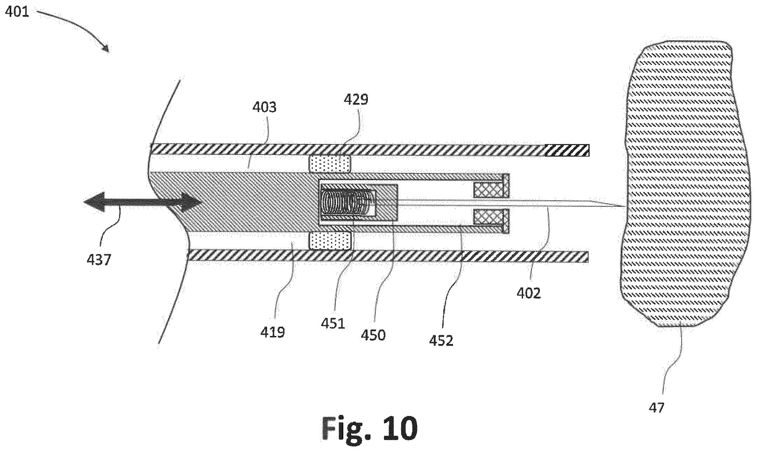

A fourth embodiment of a surgical device 401 is shown in figures 9 and 10. The surgical device 401 is similar to the surgical device 301 and includes a guide tube 403, a bearing 429, and a translational rod 419 that moves along linear motion 437. The translational rod 419 is connected to the needle 402. However, the needle 402 is not directly connected to the translational rod 419. Instead, the translational forces of the translational rod 419 and transferred via a spring 451 and piston cylinder 450 onto the needle 402. The spring 451 and the cylinder 450 are arranged within a cavity 452 of the translational rod 419. Alternatively, the spring 451 and the cylinder 450 may be simply arranged at the end of the translational rod 419. During normal operation the needle 402 follows the movement of the translational rod 419. However, in case the needle 402 collides with a hard object, the spring 451 is compressed and absorbs the excessive forces (see figure 10). Additionally, a damping element may be included in parallel or series with the spring in order to prevent unwanted oscillations of the spring 451. In some embodiments, the spring may be formed by an elastically deformable rod (e.g. a nitinol band).

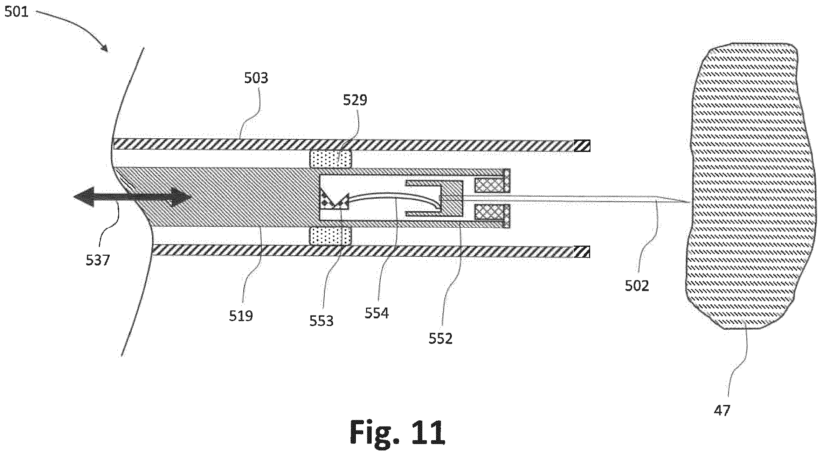

A fifth embodiment of a surgical device 501 is shown in figure 11. The surgical device 501 may be similar to the surgical device 401 shown in figures 9 and 10. However, instead of a spring, the translational rod 519 is connected to the needle 502 with a connection rod 554 and a predetermined breaking point 553. The connection rod 552 may be an elastically deformable rod that is coupled to an element featuring a predetermined breaking point 553. The predetermined breaking point 553 may be formed by means known in the art e.g. by using edges, ridges or different materials. If the needle 502 collides with bony tissue 47, the connection rod 554 is deflected. Due to the deflection, translational forces are converted to shear forces on the predetermined breaking point 553. The higher the deflection of the rod, the higher the shear forces on the predetermined breaking point 553, which is consequently more likely to break.

## Claims

1. A surgical device (1) for felting an implant to soft tissue (46) of a patient comprising at least one felting needle (2) configured to move reciprocally, and a connection interface (128; 135) for connecting the at least one felting needle to an actuator and for transferring a reciprocal motion from the interface (128; 135) to the at least one felting needle (2),
**characterized in that** the device comprises a needle protection mechanism (3; 20; 135; 242; 331; 451; 553), wherein the needle protection mechanism is configured to prevent the at least one felting needle from being damaged due to a contact with a rigid structure during the reciprocal motion, comprising a coupling (128; 235) adapted to transfer a reciprocal motion from the actuator (8) to the at least one felting needle (2) and wherein the needle protection mechanism is adapted to decouple the felting needle (2) from the actuator (8) when a threshold force or threshold moment is exceeded to prevent the felting needle from being damaged by decoupling the coupling.

2. Surgical device (1) according to claim 1, wherein the at least one felting needle has a width or diameter of less than 0.8 mm.

3. Surgical device (1) according to one the previous claims, wherein the needle protection mechanism includes a spacer (3, 20) for contacting the tissue such that the spacer sets a predetermined penetration depth for the at least one needle.

4. Surgical device (1) according to claim 3, wherein the spacer (3; 20) comprises one or more fingers (20), wherein the one or more fingers comprise a distal end surface for contacting the tissue such that the reciprocal motion of the felting needle does not exceed the predetermined penetration depth.

5. Surgical device (1) according to claim 4, wherein the spacer (20) forms a slide such that the felting needle can be slid along a surface of the soft tissue.

6. Surgical device (1) according to one the previous claims, wherein the at least one felting needle has a maximal penetration depth (16) and wherein the maximal penetration depth is adjustable.

7. Surgical device (1) according to one of the previous claims, wherein the needle protection mechanism comprises a needle collision sensor (331).

8. Surgical device (1) according to claim 7, wherein the needle collision sensor is configured to measure a bending of the at least one felting needle (2).

9. Surgical device (1) according to claim 7 or 8, wherein the surgical device comprises a controller, wherein the controller is configured to adjust the penetration depth (16) of the at least one felting needle based on measurement of the needle collision sensor (331).

10. Surgical device (1) according to one of the previous claims, wherein the needle protection mechanism includes a slipper clutch (235), wherein the slipper clutch (235) slips at least partially when the threshold force or threshold moment acting on the at least one felting needle is exceeded.

11. Surgical device (1) according to claim 10, wherein the slipper clutch includes at least one of: a pin and a chamfered face; a ball spring and a corresponding dent for receiving the ball; and a spring bearing for the at least one felting needle.

12. Surgical device (1) according to one of the previous claims, wherein the needle protection mechanism includes a predetermined breaking point wherein the predetermined breaking point breaks, if a force on the at least one felting needle exceeds a predetermined threshold.

13. System (1) comprising a surgical device according to one of the previous claims and said actuator (8) for driving the at least one felting needle with the reciprocal motion.

## Patentansprüche

1. Chirurgische Vorrichtung (1) zum Anfilzen eines Implantats an Weichgewebe (46) eines Patienten, die mindestens eine Filznadel (2), die konfiguriert ist, sich hin und her zu bewegen, und eine Verbindungsschnittstelle (128; 135) zum Verbinden der mindestens einen Filznadel mit einem Aktuator und zum Übertragen einer Hin- und Herbewegung von der Schnittstelle (128; 135) auf die mindestens eine Filznadel (2) aufweist, **dadurch gekennzeichnet, dass** die Vorrichtung einen Nadelschutzmechanismus (3; 20; 135; 242; 331; 451; 553) aufweist, wobei der Nadelschutzmechanismus konfiguriert ist, zu verhindern, dass die mindestens eine Filznadel während der Hin- und Herbewegung durch einen Kontakt mit einer starren Struktur beschädigt wird, der eine Kopplung (128; 235) aufweist, die eingerichtet ist, eine Hin- und Herbewegung von dem Aktuator (8) auf die mindestens eine Filznadel (2) zu übertragen, und wobei der Nadelschutzmechanismus eingerichtet ist, die Filznadel (2) von dem Aktuator (8) zu entkoppeln, wenn eine Schwellenkraft oder ein Schwellenmoment überschritten wird, um durch Entkoppeln der Kopplung zu verhindern, dass die Filznadel beschädigt wird.

2. Chirurgische Vorrichtung (1) nach Anspruch 1, wobei die mindestens eine Filznadel eine Breite oder einen Durchmesser von weniger als 0,8 mm aufweist.

3. Chirurgische Vorrichtung (1) nach einem der vorhergehenden Ansprüche, wobei der Nadelschutzmechanismus einen Abstandshalter (3, 20) aufweist, der das Gewebe so berührt, dass der Abstandshalter eine vorbestimmte Eindringtiefe für die mindestens eine Nadel festlegt.

4. Chirurgische Vorrichtung (1) nach Anspruch 3, wobei der Abstandshalter (3; 20) einen oder mehrere Finger (20) aufweist, wobei der eine oder die mehreren Finger eine distale Endfläche zum Kontaktieren des Gewebes aufweisen, so dass die Hin- und Herbewegung der Filznadel die vorbestimmte Eindringtiefe nicht überschreitet.

5. Chirurgische Vorrichtung (1) nach Anspruch 4, wobei der Abstandshalter (20) ein Gleitstück bildet, so dass die Filznadel entlang einer Oberfläche des Weichgewebes geschoben werden kann.

6. Chirurgische Vorrichtung (1) nach einem der vorhergehenden Ansprüche, wobei die mindestens eine Filznadel eine maximale Eindringtiefe (16) aufweist und wobei die maximale Eindringtiefe einstellbar ist.

7. Chirurgische Vorrichtung (1) nach einem der vorhergehenden Ansprüche, wobei der Nadelschutzmechanismus einen Nadelkollisionssensor (331) aufweist.

8. Chirurgische Vorrichtung (1) nach Anspruch 7, wobei der Nadelkollisionssensor konfiguriert ist, eine Biegung der mindestens einen Filznadel (2) zu messen.

9. Chirurgische Vorrichtung (1) nach Anspruch 7 oder 8, wobei die chirurgische Vorrichtung eine Steuerung aufweist, wobei die Steuerung konfiguriert ist, die Eindringtiefe (16) der mindestens einen Filznadel basierend auf der Messung des Nadelkollisionssensors (331) einstellt.

10. Chirurgische Vorrichtung (1) nach einem der vorherigen Ansprüche, wobei der Nadelschutzmechanismus eine Rutschkupplung (235) aufweist, wobei die Rutschkupplung (235) zumindest teilweise rutscht, wenn die Schwellenkraft oder das Schwellenmoment, das oder die auf die mindestens eine Filznadel wirkt, überschritten wird.

11. Chirurgische Vorrichtung (1) nach Anspruch 10, wobei die Rutschkupplung mindestens eines aufweist von: einen Stift und eine abgeschrägte Fläche; eine Kugel-Feder und eine entsprechende Vertiefung zur Aufnahme der Kugel; und ein Federlager für die mindestens eine Filznadel.

12. Chirurgische Vorrichtung (1) nach einem der vorherigen Ansprüche, wobei der Nadelschutzmechanismus eine Sollbruchstelle aufweist, wobei die Sollbruchstelle bricht, wenn eine Kraft auf die mindestens eine Filznadel einen vorgegebenen Schwellenwert überschreitet.

13. System (1), das eine chirurgische Vorrichtung nach einem der vorherigen Ansprüche und den Aktuator (8) zum Antreiben der mindestens einen Filznadel mit der Hin- und Herbewegung aufweist.

## Revendications

1. Dispositif chirurgical (1) pour le feutrage d'un implant sur un tissu mou (46) d'un patient, comprenant au moins une aiguille de feutrage (2) prévue pour se déplacer en va-et-vient, et une interface de connexion (128 ; 135) pour connecter ladite au moins une aiguille de feutrage à un actionneur et pour transférer un mouvement en va-et-vient de l'interface (128 ; 135) à ladite au moins une aiguille de feutrage (2),
**caractérisé en ce que** ledit dispositif comprend un mécanisme de protection d'aiguille (3 ; 20 ; 135 ; 242 ; 331; 451 ; 553), ledit mécanisme de protection d'aiguille étant prévu pour empêcher ladite au moins une aiguille de feutrage d'être endommagée par contact avec une structure rigide pendant le mouvement en va-et-vient, comprenant un accouplement (128 ; 235) adapté pour transférer un mouvement en va-et-vient de l'actionneur (8) à ladite au moins une aiguille de feutrage (2), et ledit mécanisme de protection d'aiguille étant adapté pour découpler l'aiguille de feutrage (2) de l'actionneur (8) lorsqu'une force seuil ou un moment couple seuil sont dépassés, afin d'empêcher l'aiguille de feutrage d'être endommagée en découplant le couplage.

2. Dispositif chirurgical (1) selon la revendication 1, où ladite au moins une aiguille de feutrage a une largeur ou un diamètre inférieur à 0,8 mm.

3. Dispositif chirurgical (1) selon l'une des revendications précédentes, où le mécanisme de protection d'aiguille comprend une pièce d'écartement (3, 20) destinée à entrer en contact avec le tissu, de manière à définir par la pièce d'écartement une profondeur de pénétration prédéterminée pour ladite au moins une aiguille.

4. Dispositif chirurgical (1) selon la revendication 3, où la pièce d'écartement (3 ; 20) comprend un ou plusieurs doigts (20), ledit un ou plusieurs doigts présentant une surface d'extrémité distale pour entrer en contact avec le tissu de sorte que le mouvement en va-et-vient de l'aiguille de feutrage ne dépasse pas la profondeur de pénétration prédéterminée.

5. Dispositif chirurgical (1) selon la revendication 4, où la pièce d'écartement (20) forme une glissière permettant à l'aiguille de feutrage d'être glissée le long d'une surface du tissu mou.

6. Dispositif chirurgical (1) selon l'une des revendications précédentes, où ladite au moins une aiguille de feutrage présente une profondeur de pénétration maximale (16) et où ladite profondeur de pénétration maximale est réglable.

7. Dispositif chirurgical (1) selon l'une des revendications précédentes, où le mécanisme de protection d'aiguille comprend un capteur de collision d'aiguille (331).

8. Dispositif chirurgical (1) selon la revendication 7, où le capteur de collision d'aiguille est prévu pour mesurer une flexion de ladite au moins une aiguille de feutrage (2).

9. Dispositif chirurgical (1) selon la revendication 7 ou la revendication 8, où ledit dispositif chirurgical comprend un contrôleur, où ledit contrôleur est prévu pour régler la profondeur de pénétration (16) de ladite au moins une aiguille de feutrage sur la base d'une mesure du capteur de collision d'aiguille (331).

10. Dispositif chirurgical (1) selon l'une des revendications précédentes, où le mécanisme de protection d'aiguille comprend un embrayage à glissement (235), ledit embrayage à glissement (235) glisse au moins partiellement lorsque la force seuil ou le couple seuil appliqués sur ladite au moins une aiguille de feutrage sont dépassés.

11. Dispositif chirurgical (1) selon la revendication 10, où l'embrayage à glissement comprend au moins l'un des éléments suivants : une goupille et une face chanfreinée ; un ressort à bille et une entaille correspondante pour recevoir la bille ; et un palier à ressort pour ladite au moins une aiguille de feutrage.

12. Dispositif chirurgical (1) selon l'une des revendications précédentes, où le mécanisme de protection d'aiguille présente un point de rupture prédéterminé, ledit point de rupture prédéterminé subissant une rupture lorsqu'une force exercée sur ladite au moins une aiguille de feutrage dépasse un seuil prédéterminé.

13. Système (1), comprenant un dispositif chirurgical selon l'une des revendications précédentes, et l'actionneur (8) pour entraîner ladite au moins une aiguille de feutrage avec un mouvement en va-et-vient.
